# FASCICULE DE BREVET EUROPEEN

(11) **EP 0 566 461 B1**
(45) Date de publication et mention de la délivrance du brevet: **04.08.1999**
(21) Numéro de dépôt: 93400937.4
(22) Date de dépôt: 09.04.1993
(51) Int. Cl.: A61F 2/16

(54) **Implant intra-oculaire pour sac capsulaire**
Intraokulares Implantat für Kapselsack
Intraocular implant for capsular bag

(30) Priorité: 14.04.1992 FR 9204585
(43) Date de publication de la demande: 20.10.1993
(73) Titulaire: CORNEAL, F-75012 Paris (FR)
(72) Inventeur: Franceschi, François, F-13012 Marseille (FR)
(74) Mandataire: Dronne, Guy

(56) Documents cités:
- EP-A- 0 346 245
- EP-A- 0 359 381
- FR-A- 2 581 535
- US-A- 4 990 159
- US-A- 5 078 740

## Description

La présente invention concerne un implant intra-oculaire destiné à être mis en place dans le sac capsulaire.

Les implants intra-oculaires constituent des systèmes correcteurs de la vision humaine qui peuvent, dans un certain nombre de cas, se substituer à des lentilles cornéennes ou à des verres correcteurs externes. Dans d'autres cas, ces implants peuvent être combinés par exemple avec des verres correcteurs pour obtenir une correction convenable de la vision. Un implant intra-oculaire se compose essentiellement d'une partie optique de forme générale circulaire ou légèrement ovalisée qui forme le système optique correcteur proprement dit. Il comporte également une partie haptique qui sert à la mise en place et à la fixation de la partie optique à l'intérieur de l'oeil dans la position convenable.

Dans les implants intra-oculaires les plus récents, la partie haptique se compose le plus souvent de deux anses plus ou moins flexibles partant de la périphérie de la partie optique et dont les extrémités viennent en contact avec la paroi interne de l'oeil pour assurer la mise en place de la partie optique et son centrage correct par rapport à l'axe oculaire de l'oeil humain.

On peut distinguer trois types principaux d'implants intra-oculaires selon la partie interne de l'oeil dans laquelle l'implant est mis en place. On trouve d'une part des implants dits de chambre antérieure qui sont placés en avant de l'iris, des implants dits de chambre postérieure qui sont placés en arrière de l'iris et, d'autre part, des implants qui sont mis en place dans le sac capsulaire après l'enlèvement du cristallin. La présente invention concerne ce dernier type d'implants.

L'opération dite de la cataracte se pratique maintenant le plus souvent selon la technique opératoire dite de phaco-émulsification. Cette technique permet l'ablation du cristallin opaque par l'introduction dans l'oeil d'une sonde à ultrasons disposant d'une irrigation-aspiration. Par l'action combinée des ultrasons et du flux de BSS, on vient retirer le cristallin par émulsification.

Cette technique opératoire présente l'avantage par rapport aux techniques antérieures de ne nécessiter que la réalisation d'une incision de dimension réduite dans la cornée pour introduire le matériel nécessaire à cette ablation. On comprend dès lors qu'il est intéressant de disposer d'implants intra-oculaires pour sac capsulaire qui puissent être introduits à l'intérieur de l'oeil et mis en place par une incision de dimension réduite profitant ainsi de l'incision elle-même de dimension réduite suffisante pour l'ablation du cristallin.

Divers implants intra-oculaires satisfont à la condition de pouvoir être introduits par une petite incision pratiquée dans la cornée. On peut citer d'une part les implants dits à anse rigide. Dans ces implants, là partie optique présente un diamètre réduit et la conservation de la géométrie habituelle pour les anses conduit à l'obtention d'anses raides qui ont tendance à subir un phénomène de torsion plus qu'à fléchir à cause d'un phénomène d'arc-boutement produit par leur courbure particulièrement faible. De tels implants sont donc peu satisfaisants.

Pour pallier aux phénomènes d'arc-boutement décrits ci-dessus, on préfère une forme plus élancée pour les anses utilisant des courbures plus fortes. Si on obtient des anses plus satisfaisantes dans leur comportement mécanique lors de la mise en place de l'implant, il en découle un encombrement plus important de l'implant. Le chirurgien doit alors soumettre l'implant lors de son introduction à des mouvements oscillants pour passer l'implant dans l'incision de diamètre réduit.

Le document FR-A-258535 décrit un implant intra-oculaire de chambre antérieure selon le préambule de revendication indépendante.

On comprend donc que les implants disponibles susceptibles d'être introduits par une incision de dimension réduite sont tous peu satisfaisants.

Pour remédier à ces inconvénients, un objet de la présente invention est de fournir un implant intra-oculaire pour sac capsulaire qui présente un encombrement réduit lors de sa phase d'introduction à l'intérieur de l'oeil tout en assurant un positionnement correct et stable de l'implant à l'intérieur du sac capsulaire.

Pour atteindre ce but, l'implant intra-oculaire selon l'invention qui comporte une partie optique de forme générale circulaire et deux anses de fixation comportant chacune une première extrémité de raccordement à la partie optique et une extrémité libre, lesdites extrémités de raccordement étant sensiblement diamétralement opposées, se caractérise en ce que chaque anse comporte entre ladite extrémité de raccordement et l'extrémité libre une zone intermédiaire présentant une plus grande largeur dans un plan sensiblement confondu avec le plan de la partie optique, et en ce que ladite extrémité de raccordement et ladite extrémité libre présentent également une plus grande largeur que le reste de l'anse, par quoi lesdites extrémités et ladite zone intermédiaire présentent une inertie de flexion dans le plan de la partie optique supérieure à celle du reste de chaque anse.

On voit que, grâce à la présence de. trois zones de largeur plus importante, on définit dans chaque anse deux zones privilégiées de flexion qui permettent, d'une part, l'adaptation de la partie de l'anse proche de son extrémité libre au contour interne du sac capsulaire et, d'autre part, une deuxième zone privilégiée de flexion entre la zone intermédiaire et la zone de raccordement qui permet d'assurer effectivement la fixation de l'implant par déformation élastique dans le sac capsulaire tout en s'adaptant aux dimensions effectives dudit sac.

De préférence, chaque anse présente à ses extrémités, respectivement libre et de raccordement, et dans sa zone intermédiaire une épaisseur selon une direction perpendiculaire au plan de la partie optique qui est inférieure à celle du reste de l'anse, par quoi chaque anse présente une inertie de torsion sensiblement constante sur toute sa longueur.

Grâce à ce mode préféré de réalisation, on évite les effets de torsion qui pourraient être induits dans les anses lors de la mise en place de l'implant dans le sac capsulaire du fait des variations de largeur de l'anse.

D'autres caractéristiques et avantages de la présente invention apparaîtront mieux à la lecture de la description qui suit d'un mode de réalisation de l'invention donné à titre d'exemple non limitatif. La description se réfère aux figures annexées sur lesquelles :
- la figure 1 est une vue en plan d'un implant intra-oculaire selon l'invention ;
- la figure 2 est une vue de côté de l'implant de la figure 1 ;
- la figure 3a représente le moment de flexion d'une anse selon sa longueur ;
- la figure 3b représente respectivement la largeur et l'épaisseur de l'anse selon sa longueur ;
- la figure 3c représente le moment de torsion de l'anse en fonction de sa longueur ; et
- la figure 3d est une vue partielle d'une anse de l'implant en perspective montrant la variation d'épaisseur et de largeur de l'anse selon le sens de sa longueur.

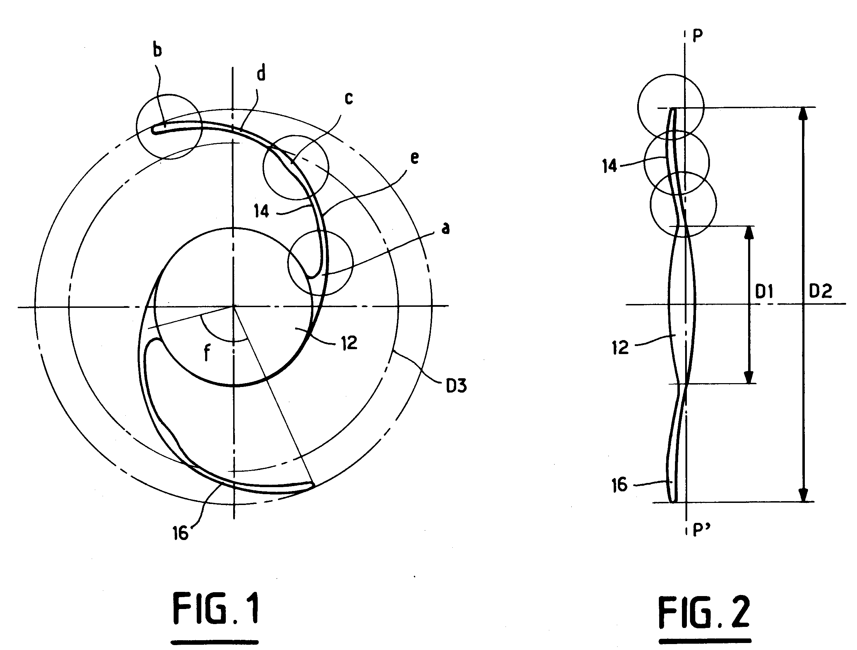

En se référant tout d'abord aux figures 1 et 2, on va décrire un mode préféré de réalisation de l'implant intra-oculaire monobloc pour sac capsulaire. Il est réalisé en un matériau transparent bio-compatible tel que le PMMA. Comme cela est bien connu, l'implant comporte une partie optique 12 de forme circulaire dans le cas de la figure 1 et une partie haptique constituée par les anses 14 et 16. Le diamètre D1 de la partie optique 12 est de 5 mm, c'est-à-dire d'une valeur relativement réduite, alors que le diamètre D2 dans lequel s'inscrivent les anses 14 et 16 en position de repos est de 12,75 mm. Dans le mode de réalisation préféré illustré par les figures 1 et 2, l'implant est du type monobloc, c'est-à-dire que la partie optique 12 et les anses 14 et 16 sont usinées dans une même pièce. Comme le montre mieux la figure 1, chaque anse 14 ou 16 comporte une extrémité de raccordement a, une extrémité libre b et une zone intermédiaire c disposée sensiblement à égale distance de l'extrémité de raccordement a et de l'extrémité libre b. Les extrémités de raccordement a sont sensiblement diamétralement opposées. De plus, on voit que chaque anse a un rayon de courbure qui garde le même signe (il n'y a pas d'inversion de courbure). La courbure de la fibre neutre de chaque anse est régulière, ce qui ne signifie pas que le rayon de courbure soit constant mais simplement que celui-ci peut varier de façon continue. Comme le montre la figure 1, les extrémités de raccordement a et libre b, ainsi que la zone intermédiaire c, présentent une largeur dans le plan PP' de l'optique qui est supérieure à la largeur du reste des anses. Ces trois portions de largeur accrue définissent entre elles deux zones d et e de largeur réduite qui présentent donc un moment de flexion réduit par rapport au moment de flexion des zones a, b et c. Sous l'effet d'une contrainte appliquée à l'anse, on comprend que l'anse va se comporter comme si elle était articulée dans les zones a et c. Sur la figure 1, les variations de la largeur de chaque anse ont été volontairement exagérées pour rendre cette figure plus lisible.

Selon un mode préféré de réalisation, la largeur de la portion d comprise entre les zones b et c est légèrement inférieure à celle de la zone e. On comprend qu'ainsi sous l'effet d'une contrainte appliquée à l'extrémité libre b, on entraîne d'abord une flexion de la portion d autour de la zone c avant d'entraîner une flexion de la portion e autour du point d'articulation constituée par la zone de raccordement a. Comme le montre mieux la figure 1, les zones intermédiaires c des anses 14 et 16 sont disposées à l'intérieur d'un cercle de diamètre D3 qui est de l'ordre de 10,5 mm dans le cas de l'exemple décrit, ce diamètre correspondant sensiblement au diamètre maximal du sac capsulaire dans sa partie interne.

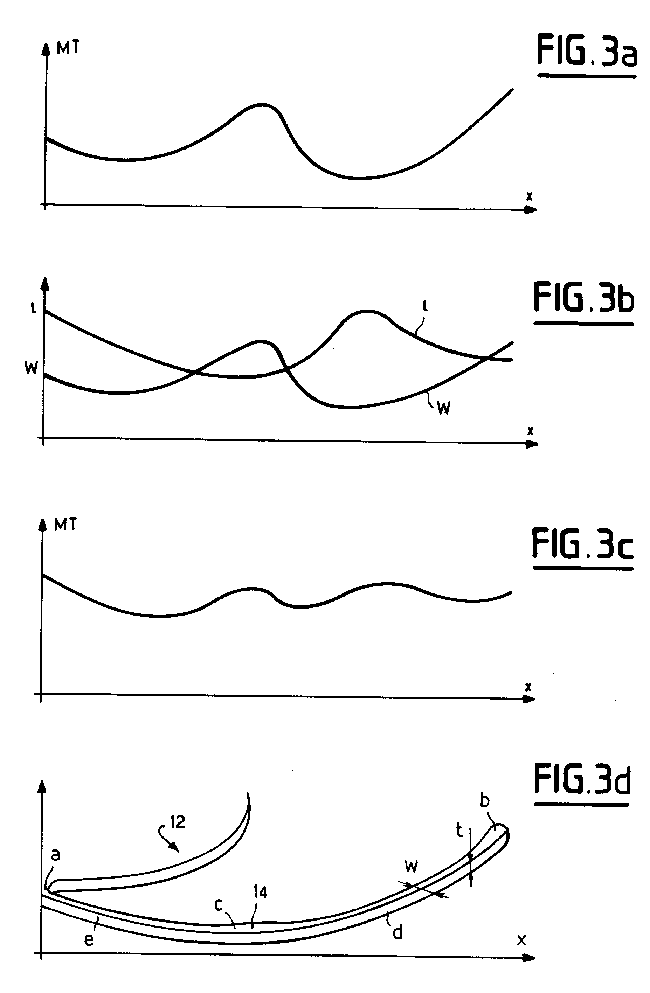

La courbe de la figure 3a représente la valeur du moment de flexion MF d'une anse en fonction de x qui représente la distance au point de raccordement de l'anse à la partie optique. On voit que, compte tenu des variations de largeur de l'anse, le moment de flexion est réduit dans les zones d et e de l'anse et encore plus réduit dans la zone d que dans la zone e..

On comprend que, en raison de la variation de la largeur w de l'anse selon sa longueur, on obtient un moment de torsion variable de cette anse, ce qui risque d'entraîner une torsion de l'anse lors de sa mise en place à l'intérieur du sac capsulaire. Pour remédier à cet inconvénient, de préférence l'épaisseur t de l'anse est également variable de telle manière que le moment de torsion de l'anse soit sensiblement constant sur toute sa longueur x en raison des variations respectives de la largeur w et de l'épaisseur t de l'anse. Plus précisément on voit que bien entendu l'épaisseur t diminue lorsque la largeur w augmente. La figure 3d montre sur un même diagramme les variations de l'épaisseur t et de la largeur w de l'anse en fonction de la longueur de l'anse x. Comme le montre la figure 3c, on obtient ainsi un moment de torsion MT qui est sensiblement constant

On voit également sur la figure 1 que l'angle au centre f qui correspond à l'anse 14 ou 16 est de l'ordre de 90' et plus précisément compris entre 80 et 110'. On comprend ainsi que l'encombrement de l'implant dans la position de repos des anses est très sensiblement réduit par rapport aux solutions à anses relativement longues de l'état de la technique. En outre, on comprend que cependant, grâce à la localisation des deux portions flexibles, on obtient une déformation convenable de chaque anse lors de sa mise en place dans le sac capsulaire évitant ainsi les phénomènes d'arc-boutement que l'on rencontre en général avec les implants à anses présentant une faible courbure et relativement rigides.

Il faut également souligner que la capacité élevée de flexion de la portion de l'anse d disposée entre l'extrémité libre b et la zone intermédiaire c est particulièrement importante puisque c'est cette portion qui vient au contact de la périphérie interne du sac capsulaire. En particulier, cette capacité localisée de flexion permet à cette portion de l'anse de s'adapter à la courbure effective du sac capsulaire dans cette région. On comprend en effet que le sac capsulaire vidé de son cristallin a tendance à s'ovaliser sous l'effet de la pression exercée par les deux extrémités libres des anses 14 et 16. Le fait que toute la portion d ou sensiblement toute la portion d soit effectivement en contact avec la périphérie interne du sac capsulaire diminue la pression locale exercée par cette anse sur la paroi interne du sac capsulaire et évite ainsi qu'il ne se développe des tissus autour de cette extrémité de l'anse comme cela se rencontre avec d'autres types d'implant. On comprend qu'avec ce développement des tissus biologiques sur l'anse l'extrémité de celle-ci serait noyée dans les tissus, ce qui rendrait très délicate l'extraction de l'implant lorsque celle-ci devient nécessaire.

En outre, le fait que les extrémités libres b des anses aient une largeur plus importante augmente la résistance mécanique de cette partie de l'anse, ce qui rend plus aisée et plus sûre la saisie de l'implant à l'aide des instruments chirurgicaux couramment utilisés pour la mise en place de l'implant à l'intérieur de l'oeil.

## Revendications

1. Implant intra-oculaire destiné à être mis en place dans le sac capsulaire d'un sujet, comportant une partie optique (12) de forme générale circulaire et deux anses de fixation (14, 16) comportant chacune une première extrémité de raccordement (a) à la partie optique et une extrémité libre (b) destinée à venir au contact du sac capsulaire, lesdites extrémités (a, b) présentant une plus grande largeur (w) dans un plan sensiblement confondu avec le plan de la partie optique que les zones voisines de ces extrémités (d, e) de chaque anse (14, 16), lesdites extrémités de raccordement (a) étant sensiblement diamètralement opposées, chaque anse présentant sur toute la longueur un rayon de courbure dont le signe ne change pas,
ledit implant se caractérisant
en ce que chaque anse comporte entre ladite extrémité de raccordement (a) et l'extrémité libre (b) une zone unique intermédiaire (c) présentant également une plus grande largeur (w) dans un plan sensiblement confondu avec le plan de la partie optique, ladite zone intermédiaire, au repos, étant disposée à l'intérieur d'un cercle concentrique à la partie optique dont le diamétre (D3) correspond à celui d'un sac capsulaire,
et en ce que lesdites extrémités et ladite zone intermédiaire présentent une inertie de flexion dans le plan de la partie optique supérieure à celle du reste de chaque anse (d, e) obtenue par la plus grande largeur (w).

2. Implant selon la revendication 1, caractérisé en ce que chaque anse présente à ses extrémités (a, b) et dans sa zone intermédiaire (c) une épaisseur (t), selon une direction perpendiculaire au plan de la partie optique, qui est inférieure à celle du reste de l'anse, par quoi chaque anse (14, 16) présente une inertie de torsion sensiblement constante sur toute sa longueur.

3. Implant selon l'une quelconque des revendications 1 et 2, caractérisé en ce qu'il est monobloc.

4. Implant selon l'une quelconque des revendications 1 à 3, caractérisé en ce que le diamètre (D1) de la partie optique (12) est de l'ordre de 5 mm.

5. Implant selon l'une quelconque des revendications 1 à 4, caractérisé en ce que la fibre neutre de chaque anse (14) présente un rayon de courbure continu.

6. Implant selon l'une quelconque des revendications 1 à 5, caractérisé en ce que, lorsque les anses sont au repos, les extrémités libres (b) des anses (14, 16) sont disposées sur un cercle dont le diamètre (D2) est de l'ordre de 12,75 mm.

7. Implant selon la revendication 6, caractérisé en ce que, lorsque les anses (14, 16) sont au repos, lesdites zones intermédiaires (c) des anses sont disposées à l'intérieur d'un cercle de diamètre (D3) de l'ordre de 10,5 mm).

8. Implant selon l'une quelconque des revendications 1 à 7, caractérisé en ce que la portion (d) de chaque anse (14, 16) comprise entre son extrémité libre (b) et la zone intermédiaire (c) présente un moment de flexion inférieur à celui de la portion (e) de l'anse comprise entre l'extrémité de raccordement (a) et la zone intermédiaire (c).

9. Implant selon l'une quelconque des revendications 1 à 8, caractérisé en ce que l'angle au centre (f) qui intercepte chaque anse (14, 16) au repose est compris entre 80 et 110 degrés.

## Claims

1. Intraocular implant intended to be placed in the capsular bag of a subject, and comprising an optical portion (12), which is circular in general shape, and two fixing loops (14, 16) each comprising a first end (a) for connecting to the optical portion and a free end (b) intended to come into contact with the capsular bag, said ends (a, b) presenting a larger width (w) in a plane roughly merged with the plane of the optical portion than the adjacent zones of these ends (d, e) of each loop (14, 16), said connecting ends (a) being more or less diametrically opposite, each loop presenting over its entire length a radius of curvature whose sign does not change,
said implant being characterised
in that each loop includes between said connecting end (a) and the free end (b) a single intermediate zone (c) which likewise presents a greater width (w) in a plane roughly merged with the plane of the optical portion, said intermediate zone, at rest, being disposed within a circle concentric with the optical portion whose diameter (D3) corresponds to that of a capsular bag,
and in that said ends and said intermediate zone present a bending inertia in the plane of the optical portion greater than that of the rest of each loop (d, e) obtained by the greater width (w).

2. Implant according to claim 1, characterised in that each loop presents at its ends (a, b) and in its intermediate zone (c) a thickness (t), in a direction perpendicular to the plane of the optical portion which is less than that of the rest of the loop, as a result of which each loop (14,16) presents a torsion inertia which is more or less constant over its entire length.

3. Implant according to any one of claims 1 and 2, characterised in that it is of one piece.

4. Implant according to any one of claims 1 to 3, characterised in that the diameter (D1) of the optical portion (12) is of the order of 5 mm.

5. Implant according to any one of claims 1 to 4, characterised in that the neutral fibre of each loop (14) presents a continuous radius of curvature.

6. Implant according to any one of claims 1 to 5, characterised in that, when the loops are at rest, the free ends (b) of the loops (14, 16) are disposed on a circle whose diameter (D2) is of the order of 12.75 mm.

7. Implant according to claim 6, characterised in that, when the loops (14, 16) are at rest, said intermediate zones (c) of the loops are disposed inside a circle whose diameter (D3) is of the order of 10.5 mm.

8. Implant according to any one of claims 1 to 7, characterised in that the portion (d) of each loop (14, 16) between its free end (b) and the intermediate zone (c) presents a bending moment lower than that of portion (e) of the loop between the connecting end (a) and the intermediate zone (c).

9. Implant according to any one of claims 1 to 8, characterised in that the centre angle (f) which intercepts each loop (14, 16) at rest is between 80 and 110 degrees.

## Patentansprüche

1. Intraokularimplantat zum Einsetzen im Kapselsack eines Menschen, das ein optisches Teil (12) mit im allgemeinen kreisförmiger Form und zwei Befestigungsbügel (14, 16) aufweist, die jeweils ein erstes Ende zur Verbindung (a) am optischen Teil und ein freies Ende (b) aufweisen, das dazu bestimmt ist, mit dem Kapselsack in Kontakt zu kommen, wobei die Enden (a, b) eine größere Breite (w) in einer Ebene, die im wesentlichen mit der Ebene des optischen Teils übereinstimmt, aufweisen als die diesen Enden benachbarten Bereiche (d, e) jedes Bügels (14, 16), wobei die Verbindungsenden (a) im wesentlichen diametral entgegengesetzt sind, wobei jeder Bügel auf der gesamten Länge eine Krümmungsradius aufweist, dessen Vorzeichen sich nicht ändert,
wobei das Implantat dadurch gekennzeichnet ist,
daß jeder Bügel zwischen dem Verbindungsende (a) und dem freien Ende (b) einen einzigen Zwischenbereich (c) aufweist, der auch eine größere Breite (w) in einer Ebene, die im wesentlichen mit der Ebene des optischen Teils übereinstimmt, aufweist, wobei der Zwischenbereich, in Ruhestellung, im Inneren eines konzentrisch zum optischen Teil verlaufenden Kreises liegt, dessen Durchmesser (D3) demjenigen eines Kapselsacks entspricht,
und daß die Enden und der Zwischenbereich eine Biegeträgheit in der Ebene des optischen Teils aufweisen, die größer ist als diejenige des Rests jedes Bügels (d, e), erhalten durch die größere Breite (w).

2. Implantat nach Anspruch 1, dadurch gekennzeichnet, daß jeder Bügel an seinen Erden (a, b) und in seinem Zwischenbereich (c ) eine Dicke (t) aufweist, in einer Richtung senkrecht zur Ebene des optischen Teils, die kleiner ist als diejenige des Rests des Bügels, wodurch jeder Bügel (14, 16) eine im wesentlichen auf seiner gesamten Länge konstante Torsionsträgheit aufweist.

3. Implantat nach einem der Ansprüche 1 und 2, dadurch gekennzeichnet, daß es in Einblockbauweise ist.

4. Implantat nach einem der Ansprüche 1 bis 3, dadurch gekennzeichnet, daß der Durchmesser (D1) des optischen Teils (12) im Bereich von 5 mm liegt.

5. Implantat nach einem der Ansprüche 1 bis 4, dadurch gekennzeichnet, daß die neutrale Faser jedes Bügels (14) einen stufenlosen Krümmungsradius aufweist.

6. Implantat nach einem der Ansprüche 1 bis 5, dadurch gekennzeichnet, daß, wenn die Bügel in Ruhestellung sind, die freien Enden (b) der Bügel (14, 16) auf einem Kreis angeordnet sind, dessen Durchmesser (D2) im Bereich von 12,75 mm liegt.

7. Implantat nach Anspruch 6, dadurch gekennzeichnet, daß, wenn die Bügel (14, 16) in Ruhestellung sind, die Zwischenbereiche (c) der Bügel im Inneren eines Kreises mit Durchmesser (D2) im Bereich von 10,5 mm angeordnet sind.

8. Implantat nach einem der Ansprüche 1 bis 7, dadurch gekennzeichnet, daß der Abschnitt (d) jedes Bügels (14, 15) zwischen seinem freien Ende (b) und dem Zwischenbereich (c) ein Biegemoment aufweist, das kleiner ist als das des Abschnitts (e) des Bügels zwischen dem Verbindungsende (a) und dem Zwischenbereich (c).

9. Implantat nach einem der Ansprüche 1 bis 8, dadurch gekennzeichnet, daß der Zentrumswinkel (f), den jeder Bügel (14, 16) in Ruhestellung einnimmt, zwischen 80 und 110° beträgt.
